# EUROPEAN PATENT APPLICATION

(11) **EP 2 250 981 A1**
(43) Date of publication of application: **17.11.2010**
(21) Application number: 09160185.6
(22) Date of filing: 13.05.2009
(51) Int. Cl.: A61F 13/00, A61F 13/02, A61F 13/06, A61F 13/14

(54) **Versatile medical article**

(71) Applicant: Meditex Ltd., Nesher, 36885 Haifa (IL)
(72) Inventor: Rosen, Uriel, 36855, Nesher (IL)
(74) Representative: Messulam, Adam Clive

(57) **Abstract**

A versatile medical article is disclosed. The versatile medical article includes: (a) an elongated elastic fabric strap characterized by having a distal end and a proximal end, a distal surface and a proximal surface, in which at least a portion of the proximal surface is outwardly furnished with a textured surface of a first kind, (b) an absorbent patch attached to the proximal surface of the strap forming a pocket therebetween, (c) a hand piece attached to the proximal end of the strap, (d) at least a portion of the distal surface outwardly furnished with a textured surface of a second kind, and (e) at least a portion of the proximal surface outwardly furnished with a textured surface of a third kind at the proximal end thereof. The textured surface of the first kind and the textured surface of the second kind self-adhere upon physical adjoining with each other thereby forming self-adherent fastener, and the textured surface of the third kind is characterized by the ability to self-adhere to the proximal surface of the strap and/or to the outer surface of the absorbent patch upon physical adjoining therewith.

## Description

The present invention relates to elastic bandages and personal dressings in general and more particularly, to a versatile medical article having self-applicability characteristics.

US Patent Ser. Nos. 5342287, 5679302 and 5830496 are believed to represent the pertinent state of the art.

The present invention will be understood and appreciated more fully from the following detailed description taken in conjunction with the appended drawings in which:
**Fig. 1** is a schematic isometric view of the versatile medical article of the present invention;
**Fig. 2** is a schematic top view of the versatile medical article of the present invention;
**Fig. 3** is a schematic bottom view of the versatile medical article of the present invention;
**Figs 4 A-D** are isometric, top and side views of the container which is to be used with the medical article of the present invention, in open conformation;
**Figs 5 A-E** are isometric, top bottom and side views of the container which is to be used with the medical article of the present invention, in closed conformation;
**Figs 6-9** are representations of several examples of self-application of the versatile medical article of the present invention to various body parts by the injured himself;
**Figs 10A-B** are respectively top views of a preferred embodiment of the absorbent patch and the medical article of the present invention comprising a pair of such patches.

In accordance with the present invention a versatile medical article is provided as set forth in the appended claims.

Illustrative embodiments of the invention are described below. In the interest of clarity, not all features of an actual implementation are described in this specification. It will be appreciated that in the development of any such actual embodiment, numerous implementation-specific decisions must be made to achieve the developers' specific goals, such as compliance with system-related and business-related constraints, which vary from one implementation to another. Moreover, it will be appreciated that such a development effort might be complex and time-consuming, but would nevertheless be a routine undertaking for those of ordinary skill in the art having the benefit of this disclosure.

The versatile medical article of the present invention can be implemented in at least three substantive medical applications and is characterized by an enhanced efficacy of being self-applied by the injured himself, namely using solely one arm for doing so. The versatile medical article of the present invention can be used: 1 - as dressing to dress by engaging in direct physical contact with saturated or excreting wounds, superficial or deep cuts, abrasions, and any other secreting wounds resulting with bleeding; 2 - as an elastic orthopaedic bandage to affix sprained or hurting joints and/or establish osteosynthesis in case of bone fractures; 3 - as a tourniquet by compressing a limb above a wound, cut, penetration or stump thereby arresting haemorrhage therefrom.

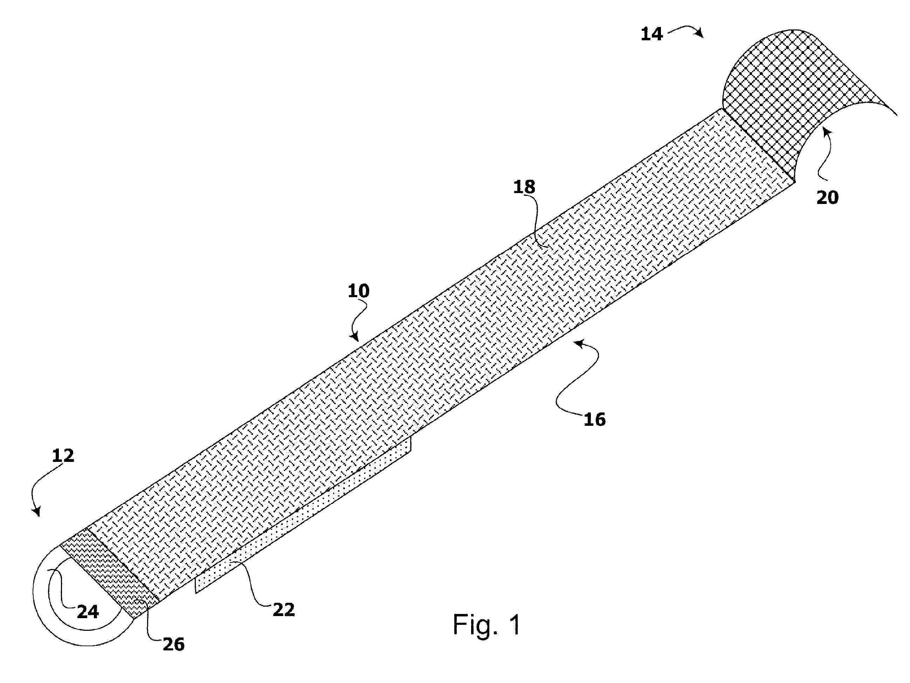

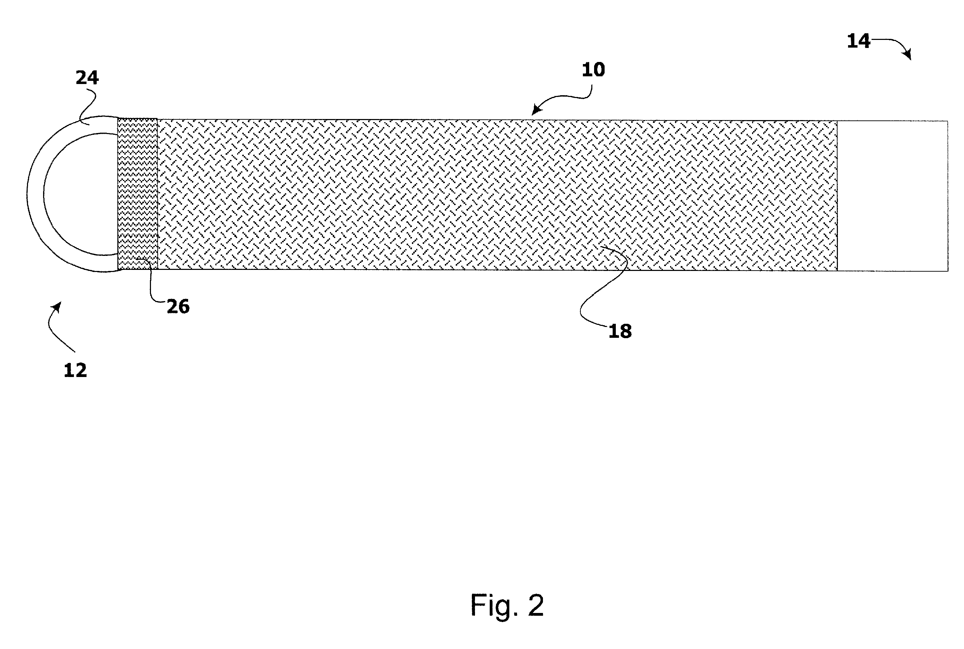

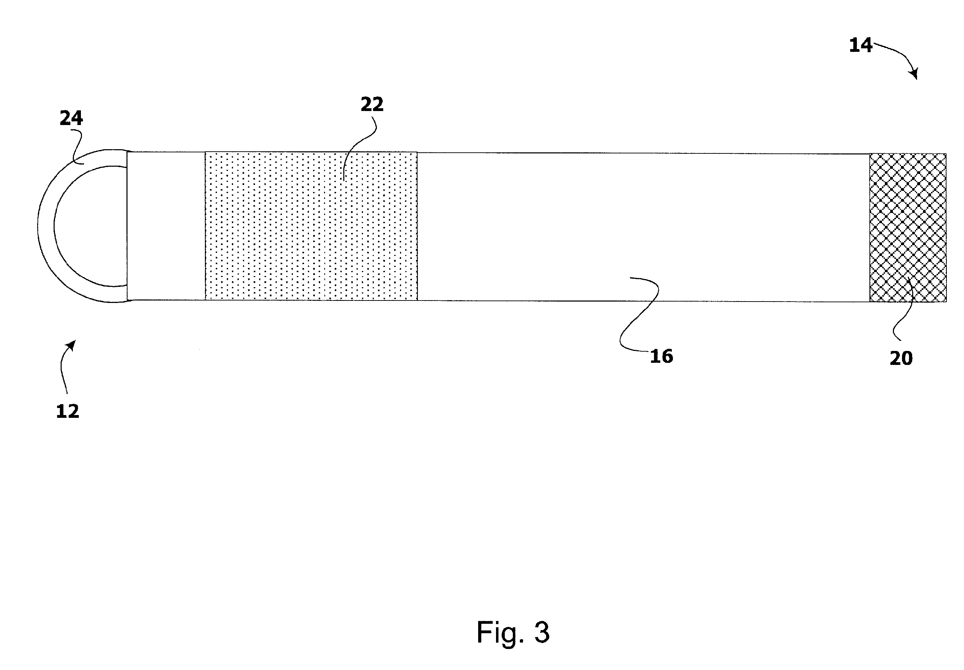

Reference is now made to Figs 1 - 3 which respectively are an isometric, top and bottom views of the versatile medical article of the present invention. Elongated elastic fabric strap 10 is characterized by having proximal end 12 and distal end 14, proximal surface 16 and distal surface 18. Distal surface 18 is superficially furnished with a textured surface of a first kind, preferably almost along the complete length thereof; whereas terminal portion 20 of proximal surface 16 is outwardly furnished with a textured surface of a second kind, preferably towards distal end 14. The textured surface of the first kind and the textured surface of the second kind self-adhere upon physical adjoining with each other thereby forming self-adherent fastener; examples of such self-adherent fasteners include the fasteners commercialized under the trade name of VELCROTM. The textured surface of the first kind and the textured surface of the second kind, as used in the present application, may refer to either the hooks or matching loops that make up self-adherent fasteners.

Absorbent patch 22 is typically made of a fibrous material to be brought into physical contact with a wound when the versatile medical article of the present invention is used as a dressing. Patch 22 may include certain chemical and biochemical modifications as will be elaborated infra. Patch 22 is attached to elastic fabric strap 10, as a result of which a pocket in-between patch 22 and strap 10 is formed; whereto a rigid object can be introduced. In some examples patch 22 has a rectangular shape and can be attached, preferably alongside longitudinal or latitudinal flanks thereof, by stitches to strap 10.

Elastic strap 10 is furnished with hand piece 24 at proximal end 12. In some preferred embodiments hand piece 24 is a strip of stretchable fabric forming a loop at proximal end 12 linking between the two corners of the butt-end of strap 10.

Portion 26 of distal surface 18 is outwardly furnished with textured surface of a third kind at proximal end 12 of elastic strap 10. The textured surface of the third kind is characterized by the ability to self-adhere to the woven proximal surface 16 or to the outer fibrous surface of absorbent patch 22 upon physical adjoining therewith. Examples of such textured surface of the third kind include mushroom-type hook strip for a mechanical fastener as disclosed in US Patent 5679302.

Whenever used as a dressing the absorbent patch of the versatile medical article of the present invention is to be engaged in direct physical contact with a saturated or excreting wound, cut, abrasion, other bleeding wound or burn. According to some embodiments of the present invention, absorbent patch may be covered, impregnated, embedded with or somehow otherwise contain either of the following agents: a super absorbent polymer (SAP); a coagulating and/or vasoconstrictive agent/s; an antiseptic, antibacterial or antifungal agent/s; anti-adhesive agents or coverings; anaesthetic or analgesic agents/s. SAP or some other applicable swelling material known in the art contained within patch absorbent, upon absorbing endogenous body fluids, such as blood that may be excreted from saturated wounds, or exogenous liquids, such as water that can be applied to patch absorbent by pouring, rinsing or injecting it thereto or immersing the patch within, is to swell and increase its bulkiness thereby facilitating an enhanced counterpressure on the wound and reducing the haemorrhage. Coagulating and/or vasoconstrictive agents, the latter include the examples of angiotensin and vasopressin, or any other of numerous suitable substances known in the art, are to biochemically modify the blood or the surrounding biological tissue wherefrom it secreted to intrinsically reduce the haemorrhage. Antiseptic, antibacterial or antifungal agents include numerous examples of suitable substances known in the art that are capable to prevent or reduce a bacterial or fungal infection of the wound. Anaesthetic or analgesic agents can be any of the numerous suitable substances known in the art that provide a relief from the pain and discomfort that may be experienced by the injured. Anti-adhesive agents or coverings preventing patch's adhering to the surface of saturated wounds include the example of TELFA^{™} Island Dressings, item code 7665, available from Tyco Healthcare / Kendall, 15 Hampshire Street, Mansfield, Massachusetts US.

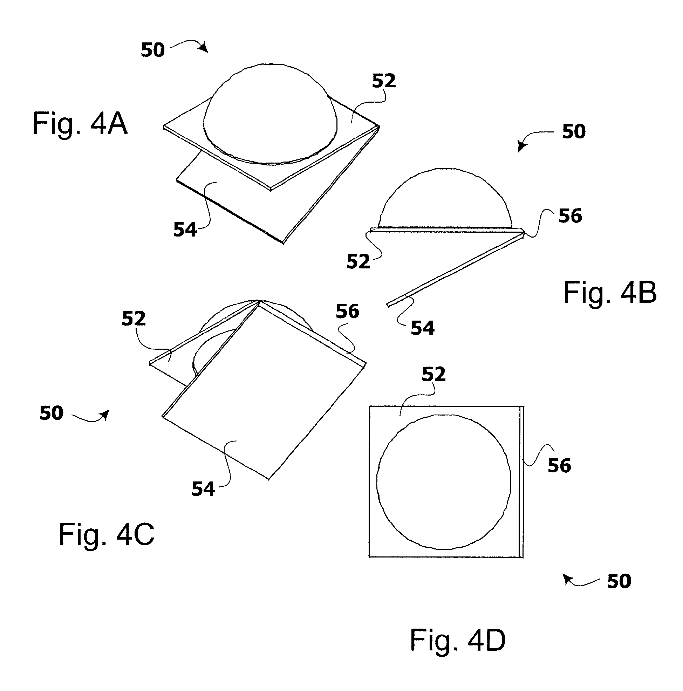

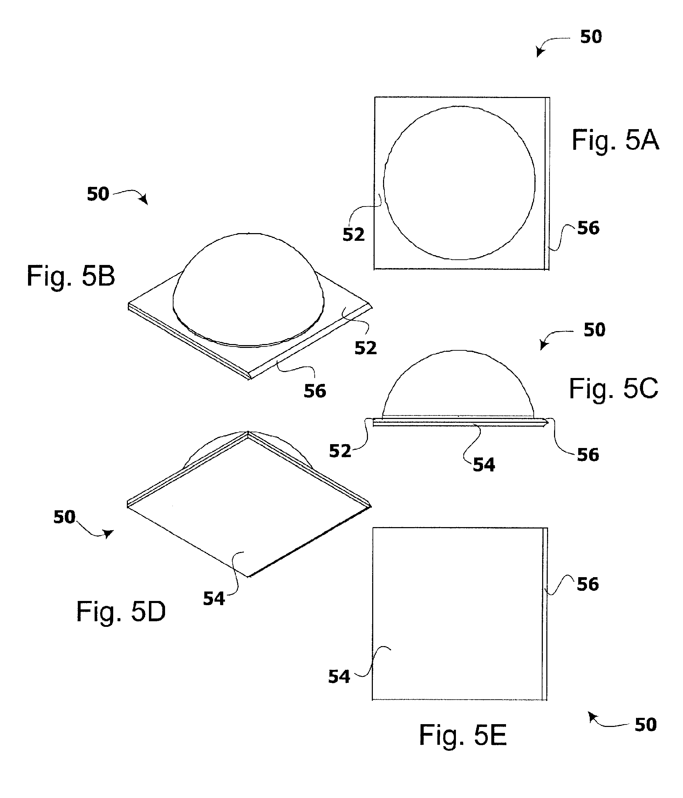

As previously mentioned a rigid object can be introduced into the pocket formed in-between patch 22 and strap 10. In some preferred embodiments, the rigid object to be introduced into the aforementioned pocket is semi-spherical container 50 illustrated in Figs 4 A-D and 5 A-E, to which reference is now made. Semi-spherical container 50 includes top and bottom base plates 52 and 54 respectively. Top base plate 52 can be shaped to outwardly form semi-spherical doom, conical or some other tapering protrusion, thereby forming between plates 52 and 54 an interior lumen of container 50. Top base plate 52 can be connected by means of hinge 56 to bottom base plate 54 and may be further furnished with a miniature latch (not shown); thereby providing for reversibly opening and closing container 50 allowing access to the interior lumen thereof. An inventory of medical items, medicaments or any other objects can be stored within the interior lumen of container 50. Container 50 or any other suitable rigid object having an apical, projecting or prominent portion can be introduced into the pocket formed in-between patch 22 and strap 10 so that the semi-spherical doom or some other tapering protrusion of container 50 or an apical, projecting or prominent portion of any other suitable rigid object is to face the wound so as to confer to the dressing a geometrical conformation that facilitates en enhanced local pressure on the wound.

In accordance with the method of the present invention, portion of the textured surface of the third kind, indicated by the numeral 26 in Figs 1 - 3 to which reference is now made again, by self-adhering to either woven proximal surface 16 of elastic fabric strap 10 or the outer fibrous surface of absorbent patch 22, provides for temporarily fastening the versatile medical article of the present invention around the body, head or limb of the injured, in the following manner. Initially hand piece 24 has to be affixed; for instance by being threaded onto a prominent or protruding portion of the body, head or limb of the injured, by being caught in injured's teeth, or by being somehow otherwise affixed. Subsequent to that, elastic strap 10, while being substantially stretched by applying tension thereto, can be circumferentially winded around the respective body part so that proximal surface 16 thereof After having circumferentially winded strap 10 around the respective body part and subsequently made proximal surface 16 thereof to self-adhered with portion 26, versatile medical article of the present invention becomes temporarily fastened and affixed around the respective body part; thereby providing for performing necessary adjustments and allowing to apply an enhanced tension to the strap during the successive windings around the respective body part fortifying the accumulative counterpressure applied thereto. After having winded the rest of strap 10 around the respective body part, terminal portion 20 of proximal surface 16 thereof, outwardly furnished with the textured surface of the second kind, is to be adjoined with distal surface 18, outwardly furnished with the textured surface of the first kind, to self-adhere therewith; thereby versatile medical article of the present invention is effectively applied to the injured. Noticeably, since the method of temporarily fastening the versatile medical article of the present invention around the respective body part, as elaborated supra, merely requires one functional arm, the capability of being self-applied by the injured himself using only one arm is provided thereby.

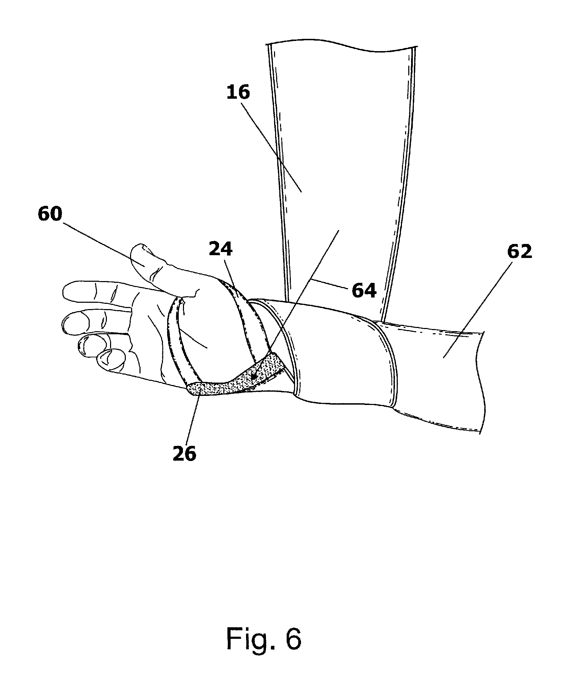

Reference is now made to Figs 6 - 9, in which several examples of self-application of the versatile medical article of the present invention by the injured himself to various body parts are shown.

In one example shown in Fig. 6, hand piece 24 is threaded onto thumb 60, the strap of the medical article is then circumferentially winded around forearm 62, proximal surface 16 thereof is brought in direction of arrow 64 and adjoined with portion 26 adhering therewith. The tension applied to the strap of the medical article can thence be enhanced and the remaining portion thereof tightly winded around forearm 62 or around the wrist area; thereby providing for effectively dressing wounds at the distal portion of forearm 62 and/or applying orthopedic bandage affixing the wrist.

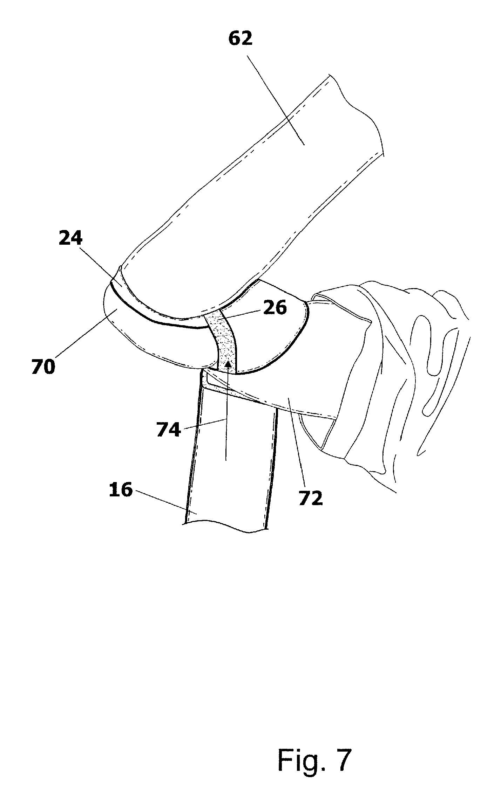

In another example shown in Fig. 7, hand piece 24 is threaded onto elbow 70, the strap of the medical article is then circumferentially winded around upper arm 72, proximal surface 16 thereof is brought in direction of arrow 74 and adjoined with portion 26 adhering therewith. The tension applied to the strap of the medical article can thence be enhanced and the remaining portion thereof tightly winded around forearm 62, upper arm 72, or around the elbow area; thereby providing for effectively dressing wounds at the proximal portion of forearm 62 and/or distal portion of upper arm 72 and/or applying orthopedic bandage affixing the wrist.

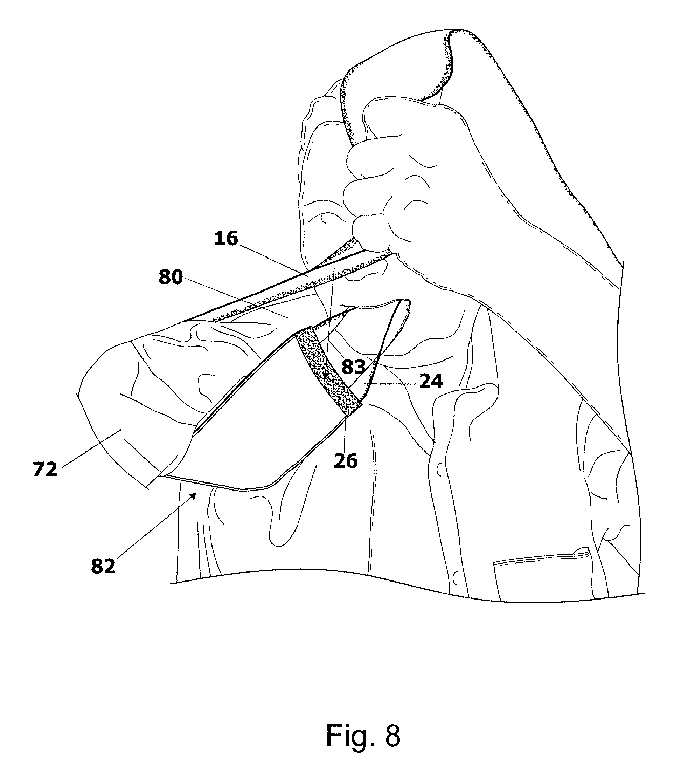

In yet another example shown in Fig 8, hand piece 24 is being caught in injured's teeth, the strap of the medical article is then winded around shoulder 80 covering axilla 82, proximal surface 16 thereof is brought in direction of arrow 83 and adjoined with portion 26 adhering therewith. The tension applied to the strap of the medical article can thence be enhanced and the remaining portion thereof tightly winded around upper arm 72 or around the shoulder; thereby providing for effectively dressing wounds at the proximal portion of upper arm 72 and/or axilla 82 and/or applying orthopedic bandage affixing the shoulder.

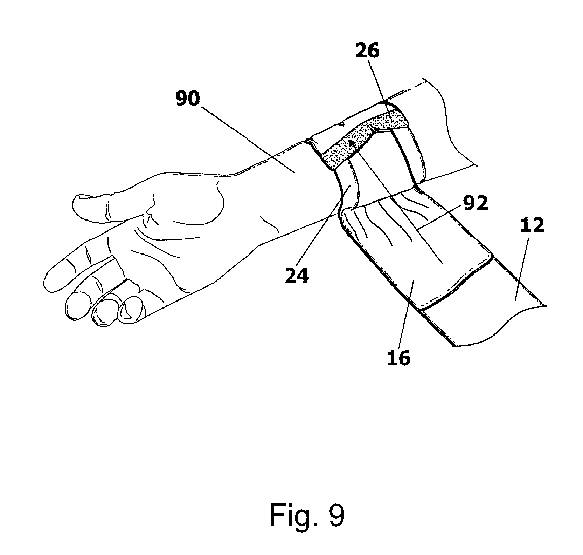

In still another example shown in Fig 9, proximal end 12 of the strap of the medical article is threaded into hand piece 24, thereby forming a loop, which can be threaded onto limb 90 or a stump thereof. Such configuration allows tightening of the article on a limb by pulling proximal end 12 outwardly therefrom. After having tightened the loop on the limb, proximal surface 16 thereof is brought in direction of arrow 92 and adjoined with portion 26 adhering therewith. The tension applied to the strap of the medical article can thence be enhanced and the remaining portion thereof tightly winded around limb 90. Present application of the article to a limb is particularly beneficial when the medical article of the present invention is used as a tourniquet, for facilitating relatively enhanced compression of the limb above a wound, cut, penetration or stump thereby thereof arresting haemorrhage therefrom and/or for establishing osteosynthesis in case of bone fractures.

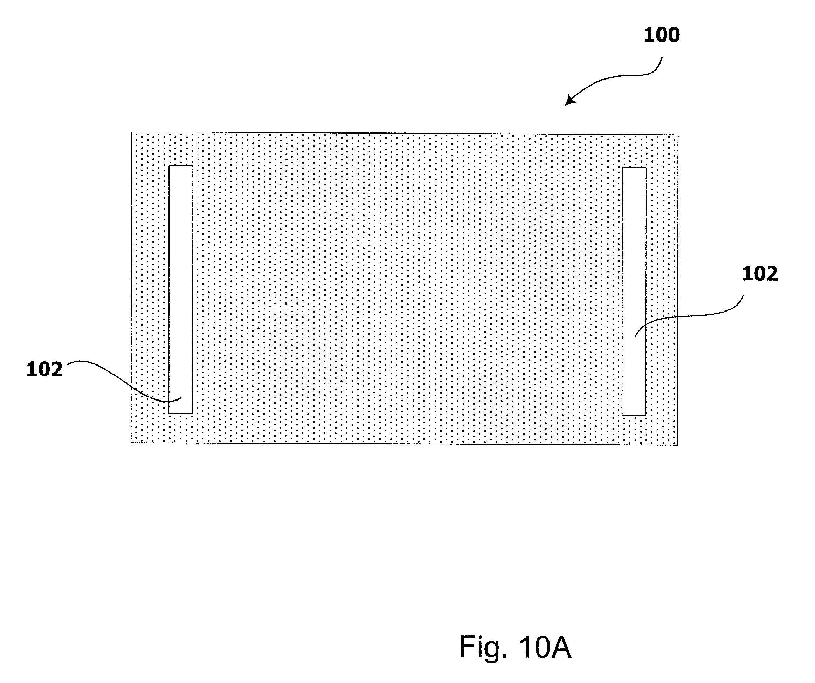

In Fig. 10A a top view of alternative preferred embodiment of patch 100 is shown. In this embodiment patch 100 is wider than elastic fabric strap 10. Patch 100 comprises at least one and preferably two latitudinal slots 102. Latitudinal slots 102 are adjusted to the wideness of elastic fabric strap 100, thus enabling threading of the patch 100 onto elastic fabric strap 10. Threaded patch 100 can be moved longitudinally along elastic fabric strap 10. The movement of threaded patch 100 along the elastic fabric strap 10 is preferably enabling patch 100 to be placed on either side of strap 10 and/or in any location along elastic fabric strap 10.

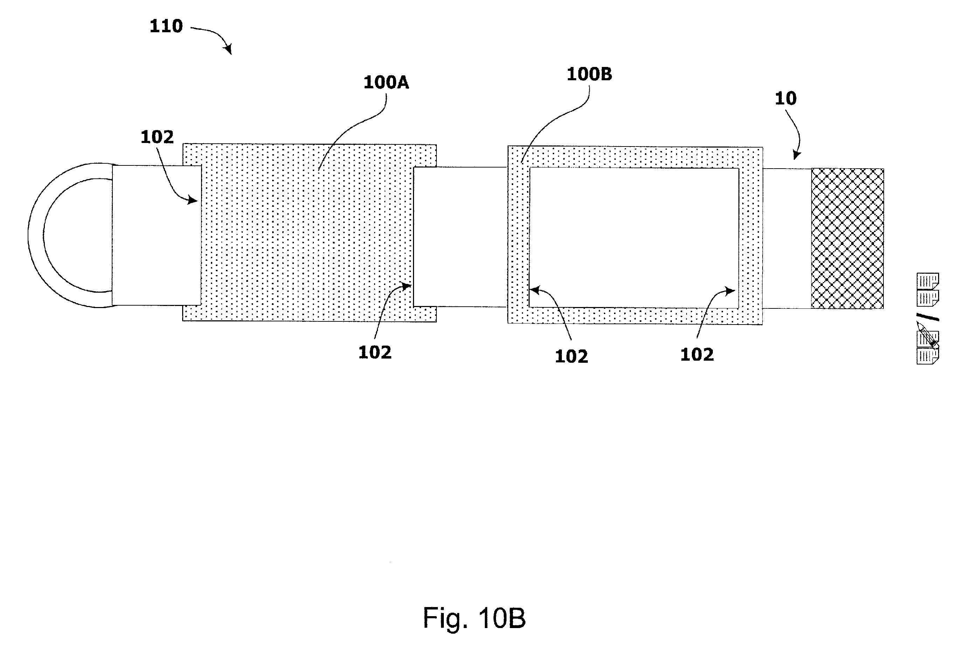

In Fig. 10B, a top view of alternative preferred embodiment 110 of the versatile medical article of the present invention is shown. In embodiment 110 a pair of patches 100A and 100B are threaded onto strap 10. In Fig. 10B, patches 100A and 100B are threaded onto strap 10 facing mutually opposite directions. It should be acknowledged, however, that the aforementioned orientation of patches 100A and 100B facing mutually opposite directions is merely exemplary; whereas typically patches 100A and 100B are threaded onto strap 10 facing mutually the same direction. It should be further acknowledged that the location of patches 100A and 100B along strap 10 is also merely exemplary and that patches 100A and 100B can be located anywhere along the entire lengths of strap 10.

In some examples, patches 100A and 100B are threaded onto strap 10 one on top of the other, thus forming a double-layered patch comprising patches 100A and 100B piled on top of each other and having enhanced absorbent capabilities. Patches 100A and 100B are typically threaded onto strap 10 facing the same direction but not on top of each other, namely located at different locations along the lengths of strap 10, in order to dress both entrance and exit wounds by a single versatile article of the invention.

According to some embodiments the method of the present invention two or more articles of the present invention can be used in a segmental manner, by successively connecting two or more articles one to another. The terminal portion of the proximal surface of the successive article, outwardly furnished with the textured surface of the second kind, is adjoined with the distal surface of the preceding article, outwardly furnished with the textured surface of the first kind, adhering therewith; thereby a segmental prolonged medical article is formed comprising two substantive articles of the present invention, which can be particularly beneficial to dress wounds around larger body parts, such as torso, and/or to be used as a tourniquet facilitating relatively enhanced compression of a limb, and/or for establishing osteosynthesis in case of bone fractures.

## Claims

1. A versatile medical article comprising:
an elongated elastic fabric strap **characterized by** having a distal end and a proximal end, a distal surface and a proximal surface; wherein at least a portion of said proximal surface is superficially furnished with a textured surface of a first kind;
an absorbent patch attached to said proximal surface of said strap; thereby forming a pocket between said strap and said patch;
a hand piece attached to said proximal end of said strap;
a textured surface of a second kind superficially covering at least a portion of said distal surface;
a textured surface of a third kind superficially covering a portion of said proximal surface at least towards said proximal end thereof;
wherein said textured surface of the first kind and said textured surface of the second kind adhere upon mutual physical contact , forming self-adherent fastener, and
wherein said textured surface of said third kind is **characterized by** the ability to adhere to said proximal surface of said strap and/or to the outer surface of said absorbent patch upon respective mutual physical contact.

2. The medical article as claimed in claim 1, wherein said absorbent patch comprising at least one selected from the group consisting of: a super absorbent polymer (SAP), a coagulating agent, a vasoconstrictive agent; an antiseptic agent, an antibacterial agent, an antifungal agent, an anti-adhesive agent or covering, an anaesthetic agent, an analgesic agent.

3. The medical article as claimed in claim 1 or 2, wherein said absorbent patch comprising at least a pair of latitudinal slots; whereby said patch can be threaded onto said strap.

4. The medical article as claimed in any of the claims herein, comprising at least a pair of said absorbent patches.

5. The medical article as claimed in claim 4, wherein said absorbent patches are piled on top of each other, thereby forming a double-layered patch having enhanced absorbent capabilities.

6. The medical article as claimed in claim 4, wherein said absorbent patches are located at different locations along the lengths of said strap, thereby allowing to dress both entrance and exit wounds with a single medical article.

7. A container to be used with the medical article as claimed in any preceding claim, said container comprising a top plate, wherein said top plate has a protrusion, and a bottom base wherein said bottom base plate and said top plate are operatively connected by a hinge.
